# EUROPEAN PATENT APPLICATION

(11) **EP 4 796 103 A1**
(43) Date of publication of application: **26.08.2026**
(21) Application number: 26159523.5
(22) Date of filing: 19.02.2026
(51) Int. Cl.: A61K 8/41, A61K 8/49, A61K 8/92, A61Q 5/10

(54) **A COMPOSITION FOR THE OXIDATIVE DYEING OF KERATINOUS FIBERS**

(30) Priority: 21.02.2025 DE 102025106692
(71) Applicant: Laboratoire Biosthetique Kosmetik GmbH & Co. KG, 75179 Pforzheim (DE)
(72) Inventor: Ader, Christian, 75179 Pforzheim (DE); Augenstein, Gunther, 75179 Pforzheim (DE)
(74) Representative: Keil & Schaafhausen Patentanwälte PartGmbB

(57) **Abstract**

The invention refers to a composition for the oxidative dyeing of keratinous fibers, particularly human hair, which comprises 2-methoxymethyl-p-phenylenediamine, one of its addition salts, its solvates and/or the solvates of its salts as well as at least one coupling agent chosen from 2-(1,3-benzodioxol-5-yla-mino)ethanol, one of its addition salts, its solvates and/or the solvates of its salts. Moreover, it contains at least one fatty substance comprising at least one triglyceride. The amount of triglyceride constitutes at least 50 wt.% of the total fatty substance content, whereby the at least one triglyceride has at least one unsaturated double bond in at least one of the esterified fatty acid residues.

## Description

The present invention relates to a composition for the oxidative dyeing of keratinous fibers, particularly human hair, comprising 2-methoxymethyl-p-phenylenediamine, one of its addition salts, its solvates and/or the solvates of its salts, at least one coupling agent chosen from 2-(1,3-benzodioxol-5-ylamino)ethanol, one of its addition salts, its solvates and/or the solvates of its salts and at least one fatty substance. The invention further encompasses a multi-component dyeing kit and a process for oxidative dyeing.

Cosmetic compositions based on oxidative dyes play a crucial role in coloring keratin fibers, particularly human hair, due to their vibrant hues and excellent long-term color stability. These formulations typically contain one or more precursors of oxidative dyes, known as primary intermediates or oxidation bases, along with one or more color coupling agents. When activated by an oxidant or the oxygen present in the air, these compositions are applied to hair fibers to create the desired dye.

Permanent hair coloring involves the formation of dyes within the hair fibers from nearly colorless, low molecular weight primary intermediates and coupling agents, primarily through oxidation with hydrogen peroxide under alkaline conditions. This process results in a color that is resistant to shampooing and experiences minimal fading over the six weeks before recoloring is needed due to new hair growth. The use of hydrogen peroxide not only develops the synthetic color but also allows for the lightening of the natural pigment by one or two shades. By carefully adjusting the proportions of dye precursors and peroxide, permanent colorants can achieve a range of lighter or darker shades.

Conventionally, dye precursors for achieving natural shades have included p-phenylenediamines as primary intermediates, combined with various coupling agents to expand the shade range. In a standard oxidation dye composition featuring p-phenylenediamine, resorcinol, m-aminophenol, and a m-phenylenediamine, these couplers react at a similar rate within a pH range of 9-10.

Color couplers are typically meta-bifunctional benzene derivatives that contain amino and/or hydroxy groups. The coupling reactions of p-phenylenediamine with resorcinol or 4-chlororesorcinol yield yellowish shades, while reactions with m-aminophenol or its derivatives produce red shades, and those with m-phenylenediamine and its derivatives result in blue shades. By mixing primary intermediates like p-phenylenediamine or its derivatives with couplers such as resorcinol, m-aminophenol, and m-phenylenediamine, a variety of natural shades can be achieved, making these products some of the best-selling hair color options.

However, resorcinol and its derivates have some disadvantages. One of the main concerns is that resorcinol can cause skin irritation or allergic reactions in some individuals, particularly with prolonged use or in high concentrations. Additionally, there are worries about its toxicity, especially with long-term exposure, as it can be absorbed through the skin and may have harmful effects on the liver and kidneys. Furthermore, resorcinol can be detrimental to aquatic life, raising environmental concerns regarding its use in certain products. Due to these potential health risks, the use of resorcinol is regulated in some countries, which may limit its availability or application in various formulations.

Therefore, in lately developed hair color cosmetics, p-phenylenediamine has largely been replaced by toluene-2,5-diamine and more recently by 2-methoxymethyl-p-phenylenediamine. Additionally, many companies are substituting resorcinol, primarily with hydroxyethyl-3,4-methylenedioxyaniline, either partially or completely, in response to consumer safety concerns.

Efforts to replace resorcinol and its derivatives with alternative coupler combinations include the pairing of 2-methoxymethyl-p-phenylenediamine with 3,4-dihydro-2H-1,4-benzoxazin-6-ol, as it is shown e.g., in WO 2023/073130 A1 and WO 2023/073131 A2, both in conjunction with specific surfactants.

Further, WO 2022/129394 A1 claims a resorcinol- and 4-chlororesorcinol-free combination of 2-methoxymethyl-p-phenylenediamine, 3,4-dihydro-2H-1,4-benzoxazin-6-ol, and 2-amino-5-ethylphenol for enhanced selectivity. It is well known in the color formulation industry that 2-amino-5-ethylphenol imparts yellow shades, making it a potential substitute for resorcinol or 4-chlororesorcinol, which helps close the yellow gap in the visible spectrum. However, this combination's drawback lies in the high cost of all three dye precursors, which makes it commercially unattractive.

Document US 2024/0065955 A1 discloses a combination of 2-methoxymethyl-p-phenylenediamine and hydroxyethyl-3,4-methylenedioxyaniline with a specific nonionic emulsifier to achieve uniform colorations along the length of the hair fiber.

US 12,109,291 B2 focuses on an agent for oxidative dyeing of keratinous fibers that is free of resorcinol. It combines 2-methoxymethyl-p-phenylenediamine with 2-(1,3-benzodioxol-5-ylamino)ethanol (an alternative name for hydroxyethyl-3,4-methylenedioxyaniline). The document explores combinations aimed at matching shades, using L*a*b* values to demonstrate the shade match. However, the document lacks details regarding the instrument and light source used to measure the color.

However, formulators face challenges in matching previous reference shades when using new dye precursors, as these new combinations can produce different chromophores with varying spectral properties in the keratin environment. So, formulators face challenges in matching previous reference shades when using new dye precursors, as these new combinations can produce different chromophores with varying spectral properties in the keratin environment. All the approaches discussed above have in common that a key issue is that natural shades, resulting from the combination of 2-methoxymethyl-p-phenylenediamine and hydroxyethyl-3,4-methylenedioxyaniline, often produce metameric effects. These cannot be adequately captured by measuring L*a*b* values with a single light source.

It is important to note that hydroxyethyl-3,4-methylenedioxyaniline cannot be considered a direct replacement for resorcinol, despite being viewed as a suitable alternative. One drawback of this compound is that hair colorants combining 2-methoxymethyl-p-phenylenediamine with hydroxyethyl-3,4-methylenedioxyaniline may produce an off-tone effect, which is particularly noticeable in sunlight when compared to references based on toluene-2,5-diamine and resorcinol. This new combination can result in an unnatural greenish shade that is difficult to correct by merely adjusting the proportions of the dye precursors.

An off-tone color refers to subtle variations of a particular shade. For example, "off-white" encompasses a range of shades, including ivory, white smoke, and snow. In the context of natural hair color, this term highlights the fact that artificially colored hair does not always absorb and reflect light in the same way as naturally pigmented hair. This is particularly evident when the dye precursors of a previous hair color are replaced with new ones, as the spectral properties of the resulting dyes differ. This phenomenon is believed to contribute to the off-tone colors observed in hair dyed with formulations containing 2-methoxymethyl-p-phenylenediamine and hydroxyethyl-3,4-methylenedioxyaniline.

However, for consumers, achieving their familiar natural shade is crucial, and the color should look natural under various lighting conditions. But due to the vast array of lighting sources in modern life, ensuring an exact color match can be challenging. This challenge is even greater when dye precursors are substituted with new molecules, as might occur during a product relaunch.

A phenomenon known as illuminant metamerism is widely recognized in the textile industry. This occurs when two colors, which appear identical under one light source, appear different under another. The same issue can arise when coloring hair, causing difficulties with color matching. For instance, while hair dyed in a salon may look identical under artificial lighting, it could show noticeable chromatic differences when exposed to daylight or direct sunlight.

Therefore, it is the object of the current invention to provide a composition for the oxidative dyeing of keratinous fibers, particularly human hair, for a uniform and intensive natural coloring, avoiding receiving off-tones and color reflections that are barely perceptible in diffuse light, while in direct sunlight they appear in different shades with an unwished sheen.

This task is solved with a composition with the features of claim 1.

Such an inventive composition comprises an oxidation base: 2-methoxymethyl-p-phenylenediamine, its addition salts, solvates, and/or solvates of its salts, a coupling agent 2-(1,3-benzodioxol-5-ylamino)ethanol, its addition salts, solvates, and/or solvates of its salts and at least one fatty substance.

The oxidation base, 2-methoxymethyl-p-phenylenediamine, is a primary intermediate that, upon oxidation, forms the core structure of the dye molecule. This compound ensures effective penetration into hair fibers and contributes to stable, long-lasting coloration.

2-(1,3-Benzodioxol-5-ylamino)ethanol or a derivate thereof serves as the coupling agent, reacting with the oxidation base to modify and stabilize the dye structure. This leads to the formation of a range of color shades, depending on the reaction conditions and additional dye components.

The at least one fatty substance features at least one triglyceride. The amount of triglyceride - independent whether only one triglyceride is contained or a mixture of at least two triglycerides are contained - constitutes at least 50 wt.-%, preferably 70 wt.-%, most preferable more than 90 wt.-% of the total fatty substance content and the at least one triglyceride or at least one of the contained triglycerides possesses at least one unsaturated double bond in its esterified fatty acid residues. Surprisingly it was found that the olive-green cast of shades can easily be effectively compensated in this composition containing a triglyceride.

Triglycerides, chemically known as triacylglycerols (TGA) or triacylglyceride), are esters derived from glycerol (propane-1,2,3-triol) and three fatty acids. Their general chemical formula is C₃H₅(OOCR)₃, where R represents the hydrocarbon chains of the fatty acids, which can be saturated or unsaturated.

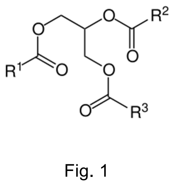

Their 2D structure is shown in figure1 wherein R1, R2 and R3 are equal or different, and stand for saturated or unsaturated fatty acid groups which can vary in length and degree of saturation, and wherein at least one group consists of an unsaturated acid group and at least one consists of a saturated acid group:

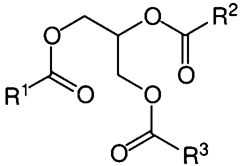

Fig. 1 Structure of a triglyceride.

It seems to be essential that only one or a mixture of several triglyceride can be part of the fatty substance, but at least there has to be a notable number of triglycerides, preferably at least 10 wt.-% of the whole composition. To ensure optimal conditioning effects, at least one triglyceride has to contain unsaturated fatty acid residues, whereby the proportion of the total mixture for the sum of the contained triglyceride(s) which have at least one unsaturated bond is preferably ate last 8 wt.-%, more preferably at least 10 wt.-%.

Beside the question regarding the shade of the colored fibers, triglycerides enhance the lipid balance of hair fibers and, therefore, reduces damage from oxidative stress during dyeing. Moreover, they improve dye spreadability and retention.

All these effects are strengthened if the at least one triglyceride or at least one triglyceride of a mixture of triglycerides is a mixed triglyceride which means that all three fatty acids substituents different from each other. Many triglycerides are known because many fatty acids are known. Nevertheless, also fatty substances containing simple triglyceride with three identical fatty acids show the described effect like stearin (derived from stearic acid), triolein (derived from oleic acid) and palmitin (derived from palmitic acid).

In a preferred embodiment, the fatty substance of the composition is a plant oil. This inclusion is significant due to the well-known benefits of plant oils in hair treatments, such as their nourishing, hydrating, and conditioning properties, which help to improve the overall health of the hair during the dyeing process. In addition, using at least one plant oil as a naturally occurring substance a positive effect on the sustainability of the product is obtained. The cosmetic composition of the present invention comprises one or more plant-derived oils. In this context, the term "oil" refers to an organic compound that is insoluble in water at room temperature (25°C) and atmospheric pressure (1013 hPa). "Insoluble" means the compound has a solubility of less than 5%, preferably less than 1%, and most preferably less than 0.1% in the specified fluid, relative to the total amount of the reference fluid. However, oils are typically soluble in many organic solvents under the same temperature and pressure conditions. Common examples of such solvents include chloroform, ethanol, and toluene.

The preferred oils in this invention are plant-derived triglycerides-fatty acid esters of glycerin-where the fatty acid moiety consists of saturated or unsaturated fatty acids with an even number of carbon atoms, ranging from C4 to C28 including the ester CO-group. Particularly preferred fatty acids include stearic acid, oleic acid, linoleic acid, linolenic acid, palmitic acid, and palmitoleic acid.

A wide range of plant oils may be selected for their varied and complementary effects on hair. These oils include, but are not limited to, almond oil, apricot kernel oil, argan oil, avocado oil, camelina oil, canola oil/rapeseed oil, castor oil, coconut oil, corn oil, cottonseed oil, cucumber seed oil, flaxseed oil, grape seed oil, hazelnut oil, hemp seed oil, linseed oil, macadamia nut oil, moringa oil, neem oil, olive oil, palm kernel oil, palm oil, peanut oil, pongamia oil, pumpkin seed oil, rice bran oil, safflower oil, sesame oil, soybean oil, sunflower oil, walnut oil and combinations thereof. Each of these oils offers unique properties-such as high levels of vitamins, antioxidants, and fatty acids-which can help protect and enhance the hair's texture while also providing moisture and shine. The specific oils selected contribute to the overall efficacy of the composition, ensuring that the dyeing process not only delivers vibrant color but also nourishes the hair fibers. Sunflower oil is particularly preferred due to its availability on the market.

Further, the composition contains certain active ingredients in preferred defined amounts. The 2-methoxymethyl-p-phenylenediamine and its addition salts, along with 2-(1,3-benzodioxol-5-ylamino)ethanol and its derivatives, are present in precise concentrations ranging from 0.001 to 7.5 weight percent. These ingredients are known to be key components in the coloration process, offering stability and durability to the color. The presence of these compounds at specific concentrations ensures optimal performance while minimizing any unwanted side effects or adverse reactions, particularly when combined with other components such as plant oils.

The total content of fatty substance(s) depends on the concentration of dye precursors. Lower dye concentrations require less oil, while higher dye concentrations necessitate more oil to compensate for off-tones. An efficient and convenient oil concentration ranges from 2 wt.-% to 20 wt.-%, with a preferred range of 3 wt.% to 15 wt.-%. The most optimal levels are 5 wt.-% for lighter shades and up to 10 wt.-% for darker shades, based on the total weight of the composition. This total amount may refer to a single fatty substance or a blend of the aforementioned fatty substances
Additionally, the composition may also incorporate additional essential components. The inclusion of alkaline agents as well as further oxidation bases/or and coupling agents enhances the effectiveness and versatility of the formulation.

Alkaline agents, such as ammonia, alkanolamines, amino acids, sodium hydroxide, or potassium hydroxide, adjust the pH of the composition to allow for better penetration of the colorants into the hair fibers. The total amount of alkaline agent in the composition ranges preferably from 0.5 to 15 wt.-% for the described effect.

In this context, acids and/or bases can be incorporated into the cosmetic composition to adjust its pH to the desired level. Suitable acids include hydrochloric acid, sulfuric acid, phosphoric acid, citric acid, maleic acid, and malic acid, as well as combinations thereof.

For alkaline adjustment, preferred agents in tint compositions include ammonia, alkanolamines (such as monoethanolamine, diethanolamine, triethanolamine, monopropanolamine, dipropanolamine, tripropanolamine, 2-amino-2-methyl-1,3-propanediol, 2-amino-2-methyl-1-propanol, and 2-amino-2-hydroxymethyl-1,3-propanediol), amino acids and their salts, guanidinium salts, alkali metal hydroxides (e.g., sodium hydroxide and potassium hydroxide), alkali metal and ammonium carbonates, and mixtures thereof. Particularly preferred are ammonia, alkanolamines, and alkali salts of amino acids.

The pH-adjusting agents typically constitute 0.1 to 20 wt.-% of the total composition, with a preferred range of 0.5 to 15 wt.-%, and an optimal concentration of 1.0 to 10.0 wt.-%. The final pH value of the cosmetic composition ranges from 6 to 12, with a more preferred range of 9.5 to 11.5. The aforementioned acids and bases are used to achieve a pH within this target range.

The primary function of an oxidation base is to serve as the precursor to the final dye molecules. The total amount of oxidation bases is between 0.001 and 10 wt.-%, preferably 0.01 to 8 wt.-%, and particularly preferably 0.05 to 5 wt.-% to ensure the described functionality.

Coupling agents play a critical role in stabilizing the color during the reaction, ensuring that the color lasts longer and appears more uniform. The total amount of coupling agents falls additionally or alternatively within the range of 0.001 to 10 wt.-% preferably 0.001 to 8 wt.-%, and particularly preferably 0.05 to 5 wt.-% for an improved stabilization.

Moreover, the optional addition of direct dyes enables the formulation to deliver more intense and precise color results. These direct dyes offer a quick-acting option for those seeking faster results, ensuring that the hair reaches the desired color intensity in a shorter time frame. The total amount of the at least one direct dye is between 0.001 and 5 wt.-% preferably in an amount of 0.005 to 4 wt.-%, and most preferably in an amount of 0.01 to 2 wt.-% to guarantee that the dyeing process is efficient, safe, and effective, delivering both aesthetic results and hair care benefits.

As discussed, the composition according to the present invention contains the primary intermediate 2-methoxymethyl-p-phenylenediamine. The total content of this oxidation base, either as the free compound or in the form of an addition salt, preferably ranges from 0.00 to 7.5wt.-%, more preferably from 0.005 to 5.0 wt.-%, relative to the total weight of the composition. Additionally, the composition may optionally include one or more additional primary intermediates distinct from the compound of formula (I) and its salts. These may be selected from p-phenylenediamines, bis(phenyl)alkylenediamines, para-aminophenols, ortho-aminophenols, heterocyclic bases, and their respective salts.

Particularly preferred additional primary intermediates include p-Phenylenediamine derivatives, such as 1,4-diaminobenzene, 1,4-diamino-2-methylbenzene, 1,4-diamino-2-(2-hydroxyethyl)benzene, 1,4-diamino-2-(3-hydroxypropyl)benzene, 1,4-diamino-2,3-dimethylbenzene, 1,4-diamino-2,6-dimethylbenzene, and 1,4-diamino-2-chlorobenzene, para-aminophenol derivatives, including 4-[di(2-hydroxyethyl)amino]aniline, 2,2'-({2-[(4-aminophenyl)amino]ethyl}imino)di-ethanol, (4-aminophenyl)-(3-(imidazol-1-yl)propyl)amine, and N,N'-bis(β-hydroxyethyl)-N,N'-bis(4'-aminophenyl)-1,3-diaminopropanol as well as other preferred compounds, such as 4-aminophenol, 4-amino-3-methylphenol, bis(5-amino-2-hydroxyphenyl)methane, 2-amino-5-ethylphenol, 1-hydroxyethyl-4,5-diamino pyrazole, 1-hexyl-4,5-diamino pyrazole, 2,3-diaminodihydroxypyrazolo pyrazolone dimethosulfonate, and 2,4,5,6-tetraaminopyrimidine.

These primary intermediates may be present as free bases or in the form of acid addition salts, such as hydrochlorides or sulfates. This simplifies the handling.

When the composition contains one or more additional oxidation bases, different from 2-methoxymethyl-p-phenylenediamine and its addition salts, their total content preferably ranges from 0.001 to 20 wt.-%, more preferably from 0.005 to 15 wt.-%, even more preferably from 0.01 to 10 wt.-%, and most preferably from 0.05 to 5 wt.-%, relative to the total weight of the composition.

The composition according to the present invention contains mandatory at least one coupler selected from 2-(1,3-benzodioxol-5-ylamino)ethanol or one of its addition salts, such as its hydrochloride salt. The total content of these s preferably ranges from 0.001 to 5.0 wt.-%, more preferably from 0.005 to 4.0 wt.-%, and most preferably from 0.01 to 3.0 wt.-%, relative to the total weight of the composition.

A broader range of natural shades can be achieved by varying dye precursors, primarily through the addition of further coupling agents, commonly used in hair color formulations. Therefore, the composition may optionally include one or more additional couplers. These additional couplers are preferably selected from compounds traditionally used for keratin fiber dyeing, including meta-aminophenols, meta-phenylenediamines, naphthols, pyridines, pyrazolones specific ortho-aminophenols (which function as self-coupling agents), indoles and indolines, along with their physiologically tolerated salts and mixtures thereof.

Particularly preferred additional coupling agents include meta-aminophenol derivatives (e.g., 3-aminophenol, 5-amino-2-methylphenol, 5-amino-4-chloro-2-methylphenol, 3-amino-2,6-dimethylphenol, 2-methyl-5-hydroxyethylaminophenol, 3-amino-2,4-dichlorophenol), benzoxazine derivatives (e.g., 3,4-dihydro-2H-1,4-benzoxazin-6-ol, 6-amino-3,4-dihydro-2H-1,4-benzoxazine), ether-linked aminophenols (e.g., 2,4-diamino-1-(2-hydroxyethoxy)benzene, 2-amino-4-[(2-hydroxyethyl)amino]anisole) bis-phenoxy derivatives (e.g., 1,3-bis(2,4-diaminophenoxy)propane), alkyl-substituted aminophenols (e.g., 1-methyl-2,6-bis-(2-hydroxyethylamino)-benzene, 2-amino-5-ethylphenol), naphthol derivatives (e.g., 1-naphthol, 2-methyl-1-naphthol, 1,5-naphthalenediol, 2,7-naphthalenediol), pyridine derivatives (e.g., 2,6-diaminopyridine, 2,6-dihydroxy-3,4-dimethylpyridine, 3,5-diamino-2,6-dimethoxypyridine, 2-amino-3-hydroxypyridine, 6-methoxy-2-methylamino-3-aminopyridine), pyrazolone derivatives (e.g., 3-methyl-1-phenyl-5-pyrazolone) and indole derivatives (e.g., 6-hydroxyindole, 5,6-dihydroxyindole).

When the composition includes one or more additional oxidation couplers, distinct from 2-(1,3-benzodioxol-5-ylamino)ethanol and its salts, their total content preferably ranges from 0.001% to 10.0% by weight, more preferably from 0.005% to 7.5% by weight, and most preferably from 0.01% to 5.0% by weight, relative to the total weight of the composition.

The hair coloring preparations according to the invention may also contain anionic, neutral, and cationic direct dyes. These dyes enhance or modify the final hair color result, providing vibrancy, depth, and tonal variation.

Particularly useful direct dyes include anionic direct dyes (e.g., acid yellow 1, acid orange 7, acid red 92, acid violet 43, tetrabromophenol blue), neutral dyes (e.g., , 2-amino-6-chloro-4-nitrophenol, 2-amino-4-nitrophenol, 2-amino-5-nitrophenol) and cationic direct dyes (basic yellow 87, basic orange 31, basic red 51, basic blue 124)
The total concentration of direct dyes in the composition typically ranges from 0.001 to 5.0 wt.-%, preferably from 0.005 to 4.0 wt.-%, and most preferably from 0.01 to 2.0 wt.-%, relative to the total weight of the composition.

The hair coloring preparations according to the invention may further comprise anionic surfactants, such as fatty alcohol sulfates, in particular sodium and ammonium lauryl sulfate, sodium cocoyl sulfate; ethoxylated fatty alcohol sulfates, in particular sodium lauryl ether sulfates with 2 to 4 units of ethylene oxide, alkylbenzene sulfonates or organic acids such as fatty acids or esters of phosphoric acid. Organic phosphate ester compounds have been commonly used for various body care products. Under the use conditions and are selected from a C₁₂ - C₂₂ monoalkyl phosphate, a monoester of phosphates of alkoxylated fatty alcohols, wherein the alkoxylated fatty alcohols are composed of C₁₂ - C₂₂ fatty alcohols alkoxylated with from 1 to 50 moles of an alkylene oxide, the number of moles of alkylene oxide with respect to the moles of fatty alcohol; and a diester of phosphates of non-alkoxylated fatty alcohols, wherein the non-alkoxylated fatty alcohols are composed of C₁₂ - C₂₂ fatty alcohols.

Some preferred combinations of the above phosphate esters are commercially available. These are often blends, e.g., Ceteth-10 phosphate und Dicetyl phosphate. Ceteth-20 phosphate and Dicetyl phosphate, Oleth-5 phosphate und Dioleyl phosphate as well as Potassium cetyl phosphate.

Examples of useful nonionic emulsifiers are for instance alkoxylated fatty alcohols such as Laureth-2, Laureth-3, Laureth-5, Laureth-8, Laureth-10 Laureth-12, Laureth-15, Laureth-20, Laureth-25, Laureth-30, Laureth-40, Laureth-50, Myreth-2, Myreth-3, Myreth-5, Myreth-8, Myreth-10, Myreth-12, Myreth-15, Myreth-20, Myreth-25, Myreth-30, Myreth-40, Myreth-50, Ceteth-2, Ceteth-3, Ceteth-5, Ceteth-8, Ceteth-10, Ceteth-12, Ceteth-15, Ceteth-20, Ceteth-25, Ceteth-30, Ceteth-40, Ceteth-50, Steareth-2, Steareth-3, Steareth-5, Steareth-8, Steareth-10, Steareth-12, Steareth-15, Steareth-20, Steareth-25, Steareth-30, Steareth-40, Steareth-50, Ceteareth-2, Ceteareth-3, Ceteareth-5, Ceteareth-8, Ceteareth-10, Ceteareth-12, Ceteareth-15, Ceteareth-20, Ceteareth-25, Ceteareth-30, Ceteareth-40, Ceteareth-50, Oleth-2, Oleth-3, Oleth-5, Oleth-8, Oleth-10, Oleth-12, Oleth-15, Oleth-20, Oleth-25, Oleth-30, Oleth-40 and Oleth-50. More preferably, Ceteth12, Ceteth-20, Steareth-12, Steareth-20, Ceteareth-12, Ceteareth-20, Polysorbate-X wherein X denotes the approximate number of ethyleneoxy units in the molecule and is equal to 20, 40, 60, 65 and 80, as well as mixtures thereof.

The total amount of surfactants is preferably in the range of from 0.1 to 30 wt.-%, preferred of from 0.5 to 15 wt.-% and most preferred of from 1.0 to 10.0 wt.-% based on total weight of the composition.

The cosmetic composition may also comprise at least one non-polymeric conditioning agent, preferably selected from cationic surfactants such as compounds commercially available under the name "quaternium," as well as quaternary surfactants like behentrimonium chloride, behentrimonium methosulfate, cetrimonium chloride, and stearalkonium chloride. These conditioning agents may be present in a concentration ranging from 0.05 to 10.0 wt.-%, preferably from 0.1 to 5.0 wt.-%, and more preferably from 0.2 to 2.0 wt.-% of the total weight of the composition.

Polyquaterniums are particularly beneficial due to their positive charges, which neutralize the negative charges of hair proteins, helping to smooth the hair and improve combability. They ionically bond to the hair, ensuring effective conditioning. The cosmetic composition may comprise cationic polymers classified as polyquaternium compounds according to INCI. Preferred polyquaternium compounds include polyquaternium-37 (poly(2-methacryloxyethyltrimethylammonium chloride)), polyquaternium-4 (hydroxyethyl cellulose dimethyl diallylammonium chloride copolymer), polyquaternium-10 (quaternized hydroxyethyl cellulose), and polyquaternium-7 (copolymer of acrylamide and diallyldimethylammonium chloride). Particularly preferred are polyquaternium-22 (copolymer of acrylic acid and diallyldimethylammonium chloride) and polyquaternium-39 (terpolymer of acrylic acid, acrylamide, and diallyldimethylammonium chloride), both containing diallyldimethylammonium chloride (DADMAC). Polyquaterniums may be present at a concentration of 0.05 to 20.0 wt.-%, preferably 0.1 to 15.0 wt.-%, more preferably 0.2 to 10.0 wt.-%, and most preferably 0.2 to 2.0 wt.-% of the total weight of the composition.

In a further preferred embodiment, the cosmetic composition may contain complex-forming agents conventionally used in hair cosmetics to bind metal ions such as iron or copper, preventing their negative effects on hair and dye stability. Suitable complex-forming agents include salicylic acid, 8-hydroxyquinoline, 1-hydroxyethane-1,1-diphosphonic acid, amino-tri-(methylene phosphonic acid), ethylenediamine tetraacetic acid (EDTA), ethylenediamine disuccinic acid (EDDS), dipicolinic acid, and their sodium salts. The total amount of chelating agents in the composition typically ranges from 0.01 to 0.5 wt.-%, based on the total weight of the composition. Due to its reduced activity and higher molecular weight, EDDS can be used at higher levels, up to 2.0 wt.-%.

To achieve the desired texture and stability, the cosmetic composition may include viscosity-regulating agents, such as thickeners. Suitable examples include agar-agar, guar gum, alginates, xanthan gum, natural rubber, Arabic gum, karaya gum, locust bean flour, linseed gums, dextrans, cellulose derivatives (e.g., methyl cellulose, hydroxyalkyl cellulose, carboxymethyl cellulose), starch derivatives (e.g., amylose, amylopectin, dextrins), clays (e.g., bentonite), and synthetic hydrocolloids (e.g., polyvinyl alcohols). The total amount of viscosity-regulating agents preferably ranges from 0.01 to 5.0 wt.-%, based on the total weight of the composition.

The composition may also contain other commonly used ingredients in hair color cosmetics, such as antioxidants, preservatives, and stabilizers. Preferred antioxidants include ascorbic acid, isoascorbic acid, sodium sulfite, sodium hydrosulfite, sodium metabisulfite, and butylated hydroxytoluene (BHT). Suitable preservatives may include benzoic acid and its salts, phenoxyethanol, parabens, or formaldehyde-releasing agents such as DMDM hydantoin. These ingredients may be present in an amount ranging from 0.0001 to 0.5 wt.-%, depending on their function, based on the total weight of the composition.

Permanent hair colorants are typically marketed as two-component kits. One component contains dye precursors, conventionally formulated in an alkaline liquid or cream base, while the other component is a stabilized acidic solution of hydrogen peroxide. These two components are mixed immediately before use and applied to the hair, where they diffuse into the hair shaft, allowing color formation to take place.

Therefore, another aspect of the invention is a kit for coloring keratin fibers, comprising at least two individually packaged components: A first component containing the dye composition as described above, particularly with the features of any of claims 1 to 13. A second component contains an oxidizing agent, preferably an aqueous hydrogen peroxide composition with an acidic pH. Most preferably the pH-value is in the range of 2.0-4.0 to ensure optimal conditions for the chemical reactions.

This kit sums up the advantages of a resorcinol-free hair dying, reducing the risk of allergic reactions associated with optimal coloration.

Moreover, the invention is also directed to a process for oxidative dyeing involving the following steps:
- mixing the kit components according to claim 14 to form a ready-to-use mixture,
- applying the mixture to keratinous fibers
- leaving the composition on the fibers for 1 to 60 minutes at room temperature or higher
- rinsing the fibers with water and/or a cleaning agent.

This provides an improved oxidative dyeing process that balances effective hair coloration with superior hair conditioning properties. By incorporating triglyceriderich fatty substances, the formulation reduces not only oxidative damage but ensure a high quality of the obtained results independent from the light source.

### Examples

The following examples illustrate various aspects of the invention. These examples and embodiments are provided for illustrative purposes only. Modifications or adaptations by those skilled in the art may be made without departing from the scope of the invention.

Unless otherwise stated, all amounts mentioned in the tables below refer to weight percentage (wt.-%). References to rinsing or washing hair with water indicate the use of lukewarm tap water. When water is a component of a composition, demineralized water was used.

L*a*b* values were measured using a Konica-Minolta Chromameter CR-400 with a D65 light source and an 8mm illumination area.

### Example 1: Tint Compositions

A composition of color cream base and developer has been examined all figures are in percent by weight unless otherwise stated):

**Table 1.1: Composition of the tint base.**

| Ingredient | Weight % |
|---|---|
| Laureth-3 | 2.00 |
| Xanthan gum | 0.60 |
| Ceteth-10 phosphate | 1.05 |
| Dicetyl phosphate | 1.05 |
| Steareth-100 | 1.00 |
| Cetearyl Alcohol | 6.90 |
| Lauryl alcohol | 1.00 |
| Myristyl alcohol | 1.00 |
| Tromethamine | 4.00 |
| Sodium sulfite | 0.20 |
| Sodium hydrosulfite | 0.20 |
| Ascorbic acid | 0.40 |
| Glycine | 4.50 |
| Betaine | 0.10 |
| EDTA | 0.20 |
| Sodium hydroxide | 2.75 |
| Ammonia (25% in water) | 3.40 |
| Dyes, glycerin, and plant oil | As per Tables 1.3a-c, and 2 |
| Water | Ad 100.00 |

**Table 1.2: Developer Composition (Hydrogen Peroxide Strength 6%).**

| Ingredient | Weight % |
|---|---|
| Demineralized Water | 80.25 |
| Hydrogen Peroxide (35% in water) | 17.15 |
| Cetearyl Alcohol | 2.00 |
| Ceteareth-50 | 0.50 |
| Phosphoric Acid (85%) | 0.10 |
| Total | 100.00 |

The pH value of the developer composition is 3.0.

### Testing Protocol: Half-Head Hair Color Assessment

A comparative assessment was conducted using the half-head test, a standard method for evaluating hair coloring properties. Two tint compositions (reference and invention) were mixed with oxidizing composition B in a 1:1.5 weight ratio.

Trained hairdressers with extensive experience in hair coloring divided test subjects' hair down the middle, applying one product to each half. After a 30-minute leave-on time, the hair was rinsed, shampooed, and dried. Hair color results were then evaluated under various lighting conditions.

The compositions and ratings are summarized in Tables 1.3a-c (all quantities in weight-%, referring to the total weight of the agent for the oxidative dyeing added to the cream base of Table 1):

**Table 1.3a: Dye Powder/Oil Formulas and Colorimetric Assessments for Shade 6/0.**

| Ingredient | 6/0 C1 (Comparison) | 6/0 C2 (Comparison) | 6/0 S (Invention) |
|---|---|---|---|
| Toluene-2,5-diamine sulfate | 1.430 | - | - |
| 2-Methoxymethyl-p-phenylenediamine | - | 1.060 | 1.060 |
| 2,4-Diaminophenoxy-ethanol HCl | 0.007 | - | - |
| m-Aminophenol | 0.077 | 0.140 | 0.140 |
| Resorcinol | 0.352 | - | - |
| 2-Methylresorcinol | 0.319 | - | - |
| Hydroxyethyl-3,4-methylene-dioxyaniline HCl | - | 1.160 | 1.160 |
| p-Aminophenol | - | 0.320 | 0.320 |
| 2-Amino-3-hydroxypyridine | - | 0.050 | 0.050 |
| Phenyl methyl pyrazolone | - | 0.500 | 0.500 |
| Glycerin | 7.500 | 7.500 | - |
| Sunflower Oil | - | - | 7.500 |
| Assessment | Reference, light brown, no unwished reflexes | Neutral light brown with greenish reflexes | Neutral light brown, identical with reference |
| Hairdressers' scoring of the undesired color deviation from the neutral target shade | Target shade | 3 | 0 |

### Hairdressers' Scoring of Color Deviation

| Score | Definition |
|---|---|
| 0 | Identical to target shade, no visible deviation |
| 1 | Slightly visible greenish reflexes |
| 2 | Moderate greenish reflexes |
| 3 | Markedly greenish reflexes |

Similar data tables for shades 10/0 and 3/0 were compiled in Tables 1.3b and 1.3c, respectively.

**Table 1.3b: Dye powder/oil formulas and colorimetric assessments for Shade 10/0 (all quantities in weight-%, referring to the total weight of the agent for the oxidative dyeing added to the cream base of Table 1).**

| **Shade 10/0** | **10/0 C1 Comparison** | **10/0 C2 Comparison** | **10/0 S Invention** |
|---|---|---|---|
| Toluene-2,5-diamine sulfate | 0.326 | - | - |
| 2-Methoxymethyl-p-phenylenediamine | - | 0.157 | 0.157 |
| m-Aminophenol | 0.012 | 0.007 | 0.007 |
| Hydroxyethyl-3,4-methylene-dioxyaniline HCl | - | 0.050 | 0.050 |
| Resorcinol | 0.077 | - | - |
| 2-Methylresorcinol | 0.084 | - | - |
| Phenyl methyl pyrazolone | - | 0.130 | 0.130 |
| Glycerin | 5.000 | 5.000 | - |
| Sunflower oil | - | - | 5.000 |
| | | | |
| Assessment | Reference, light blonde, no unwished reflexes | Light blonde with slight olive reflexes | Light blonde, identical with reference |
| Hairdressers' scoring of the undesired color deviation from the neutral target shade | Target shade | 1 | 0 |

**Table 1.3c: Dye powder/oil formulas and colorimetric assessments for Shade 3/0 (all quantities in weight-%, referring to the total weight of the agent for the oxidative dyeing added to the cream base of table 1).**

| **Shade 3/0** | **3/0 C1 Comparison** | **3/0 C2 Comparison** | **3/0 S Invention** |
|---|---|---|---|
| Toluene-2,5-diamine sulfate | 4.38 | - | - |
| 2-Methoxymethyl-p-phenylenediamine | - | 1.7833 | 1.7833 |
| N,N-Bis(2-hydroxyethyl)-p-phenylenediamine sulfate | - | 0.7533 | 0.7533 |
| p-Aminophenol | | 0.2478 | 0.2478 |
| 2,4-Diaminophenoxy-ethanol HCl | 0.0215 | 0.2083 | 0.2083 |
| m-Aminophenol | 0.46 | 0.3167 | 0.3167 |
| Hydroxyethyl-3,4-methylene-dioxyaniline HCl | - | 2.1783 | 2.1783 |
| Resorcinol | 1.275 | - | - |
| 2-Methylresorcinol | 0.500 | - | - |
| 2-Amino-3-hydroxypyridine | - | 0.0992 | 0.0992 |
| Phenyl methyl pyrazolone | - | 0.300 | 0.300 |
| Glycerin | 9.500 | 9.500 | - |
| Sunflower oil | - | - | 9.500 |
| Assessment | Reference, dark brown, no unwished reflexes | Dark brown with greenish reflexes | Dark brown, identical with reference |
| Hairdressers' scoring of the undesired color deviation from the neutral target shade | Target shade | 2 | 0 |

### Example 2: Performance Evaluation - Natural vs. Mineral Oils

To assess the effect of various oils, Sunflower Oil in Shade 6/0 S was replaced with Rapeseed Oil (Shade 6/0 R) and Mineral Oil (Shade 6/0 M).

**Table 2: Performance of Oils in Hair Dyeing**

| **Shade 6/0** | **6/0 S (Sunflower Oil)** | **6/0 R (Rapeseed Oil)** | **6/0 M (Mineral Oil)** |
|---|---|---|---|
| **Hairdressers' Scoring** | **0** | **1** | **3** |

Half-head tests were performed on model heads and rated by trained hairdressers under various light conditions. Scoring by the hairdressers indicated that Sunflower oil reduces unwished reflexes best, followed by Rapeseed oil which is somewhat less performing and lastly Mineral oil, which is practically inactive with respect to the current invention.

In the following tables L*a*b* values were taken from model heads on which shades comprising Sunflower oil were tested against the corresponding shades comprising Rapeseed oil and Mineral oil, respectively. As models' natural shades and hair structures varied a lot, a used statistical approach was used to evaluate the impact of the oils on the dye performance.

**Table 2.1: L*a*b* Values of Shade 6/0 comprising Glycerin (Comparison 6/0 C2) vs. Sunflower oil (Invention 6/0 S).**

| **Model #** | **L*a*b* 6/0 C2 (Table 3a)** | **L*a*b* 6/0 S (Table 3a)** | **Δa*** | **Δb*** |
|---|---|---|---|---|
| 1 | 51.9 1.1 11.4 | 51.4 0.0 6.1 | -1.1 | -5.3 |
| 2 | 70.8 12.0 32.3 | 72.6 8.4 22.2 | -3.6 | -10.1 |
| 3 | 65.6 11.0 26.7 | 63.1 10.5 23.8 | -0.5 | -2.9 |
| 4 | 54.6 9.8 15.8 | 54.3 8.6 15.3 | -1.2 | -0.5 |
| 5 | 37.8 13.5 20.1 | 34.4 12.0 18.4 | -1.5 | -1.7 |
| 6 | 59.2 13.4 24.7 | 58.2 12.6 22.6 | -0.8 | -2.1 |
| 7 | 60.1 13.0 18.0 | 67.6 10.8 15.6 | -2.2 | -2.4 |
| 8 | 52.2 19.5 24.2 | 54.1 17.9 23.5 | -1.6 | -0.7 |
| 9 | 64.0 6.8 14.8 | 69.1 4.6 15.5 | -2.2 | 0.7 |
| 10 | 49.7 17.9 19.5 | 49.8 11.9 13.4 | -6 | -6.1 |
| 11 | 67.3 17.7 26.0 | 71.6 12.9 18.4 | -4.8 | -7.6 |
| 12 | 43.8 18.7 19.6 | 42.8 18.9 22.1 | 0.2 | 2.5 |
| 13 | 64.9 14.8 19.7 | 61.8 10.3 17.4 | -4.5 | -2.3 |
| Average | | | -2.3 | -3.0 |

**Table 2.2: L*a*b* Values of Shade 6/0 comprising glycerin (Comparison 6/0 C2) vs. Rapeseed oil (Invention 6/0 R).**

| **Model #** | **L*a*b* 6/0 C2** | **L*a*b* 6/0 R** | **Δa*** | **Δ*** |
|---|---|---|---|---|
| 1 | 53.1 11.5 17.1 | 57.6 9.1 16.0 | -2.4 | -1.1 |
| 2 | 66.0 15.5 26.3 | 64.0 9.7 18.9 | -5.8 | -7.4 |
| 3 | 68.0 14.5 25.7 | 59.1 11.6 22.2 | -2.9 | -3.5 |
| 4 | 46.9 15.0 21.4 | 49.7 12.4 20.7 | -2.6 | -0.7 |
| 5 | 40.6 11.3 22.1 | 31.6 11.3 19.6 | 0 | -2.5 |
| 6 | 64.2 16.6 18.2 | 65.1 11.6 16.6 | -5 | -1.6 |
| 7 | 68.8 16.6 24.3 | 72.9 17.3 29.4 | 0.7 | 5.1 |
| 8 | 62.5 14.6 19.7 | 57.1 18.6 20.0 | 4 | 0.3 |
| 9 | 75.8 13.6 19.3 | 81.0 11.0 18.1 | -2.6 | -1.2 |
| 10 | 61.1 17.8 22.3 | 57.4 18.3 24.5 | 0.5 | 2.2 |
| 11 | 66.7 18.5 26.9 | 58.1 15.3 20.8 | -3.2 | -6.1 |
| 12 | 65.3 12.5 18.2 | 58.4 13.6 17.8 | 1.1 | -0.4 |
| 13 | 60.8 13.9 17.8 | 59.5 10.8 14.2 | -3.1 | -3.6 |
| 14 | 63.8 16.3 21.6 | 61.3 14.4 19.1 | -1.9 | -2.5 |
| Average | | | -1.7 | -1.6 |

**Table 2.3: L*a*b* Values of Shade 6/0 comprising Glycerin (Comparison 6/0 C2) vs. Mineral oil (Invention 6/0 M).**

| **Model #** | **L*a*b* 6/0 C2** | **L*a*b* 6/0 M** | **Δa*** | **Δb*** |
|---|---|---|---|---|
| 1 | 67.3 9.3 19.7 | 65.5 8.2 19.3 | -1.1 | -0.4 |
| 2 | 60.8 15.2 24.1 | 56.6 15.3 23.9 | 0.1 | -0.2 |
| 3 | 70.6 10.7 18.4 | 57.0 15.6 22.2 | 4.9 | 3.8 |
| 4 | 47.7 16.4 23.8 | 47.4 15.1 23.2 | -1.3 | -0.6 |
| 5 | 60.5 15.6 20.3 | 61.1 13.6 18.8 | -2 | -1.5 |
| 6 | 61.0 13.2 18.1 | 61.4 10.6 16.4 | -2.6 | -1.7 |
| 7 | 64.5 12.0 16.8 | 63.6 11.4 16.0 | -0.6 | -0.8 |
| 8 | 62.4 12.3 24.0 | 61.6 10.6 19.9 | -1.7 | -4.1 |
| 9 | 66.1 15.3 17.5 | 63.8 11.3 13.1 | -4 | -4.4 |
| 10 | 62.3 9.6 13.0 | 61.2 12.6 19.0 | 3 | 6 |
| 11 | 73.5 10.1 21.9 | 65.7 13.6 20.9 | 3.5 | -1 |
| Average | | | -0.2 | -0.4 |

The overall conclusion is that Δa* and Δb* were both significantly reduced with the addition of Sunflower oil, less significant was the effect of Rapeseed oil and almost no effect had the addition of Mineral oil. After the visual assessment of the hair under salon light and direct sunlight no metameric effects were detected in case of formulas comprising Sunflower oil. The amount of oil correlates with the amount of dye precursors; a higher dye load requires a higher amount of oil and vice versa.

### Example 3: Performance Evaluation of Plant Oils in Hair Color Compositions

Ready-to-use oxidative hair coloring agents were prepared by mixing each color cream according to Table 1.1 in combination with Table 1.3, wherein the 7.5% oil ingredient was replaced by the same amount of further natural plant oils. The tints were mixed with oxidizing agent from table 1.2 in a 1 + 1.5 parts by weight, respectively.

Each ready-to-use composition was applied to 50% grey hair strands (supplied by Kerling, Backnang, Germany) immediately after its preparation (four grams of the hair color per gram of hair) and left on the hair for 30 minutes at 30°C. After processing the hair strands were shampooed and rinsed out with water. The strands were then dried with a towel and dried with a hair dryer.

The dyed hair strands were evaluated under salon light conditions and direct sunlight. Rapeseed oil, olive oil, walnut oil, almond oil, grape seed oil, peanut oil, sesame seed oil, coconut oil provided a positive effect in reducing unwished greenish reflexes; mineral oil as commonly used oil in hair cosmetics had no significant impact. The most effective oil was sunflower oil.

## Claims

1. A composition for the oxidative dyeing of keratinous fibers, particularly human hair, comprising:
(a) 2-methoxymethyl-p-phenylenediamine, one of its addition salts, its solvates and/or the solvates of its salts,
(b) at least one coupling agent chosen from 2-(1,3-benzodioxol-5-yla-mino)ethanol, one of its addition salts, its solvates and/or the solvates of its salts,
(c) at least one fatty substance,
**characterized in that** the at least one fatty substance comprises at least one triglyceride, wherein the amount of triglyceride constitutes at least 50 wt.% of the total fatty substance content and that at least one triglyceride has at least one unsaturated double bond in at least one of the esterified fatty acid residues.

2. A composition according to claim 1, **characterized in that** the least one triglyceride is a mixed triglyceride.

3. A composition according to claim 1 or 2, **characterized in that** the at least one fatty substance is a plant oil.

4. A composition according to any of the preceding claims, **characterized in that** the fatty substance is selected from the group consisting of almond oil, apricot kernel oil, argan oil, avocado oil, camelina oil, canola oil/rapeseed oil, castor oil, coconut oil, corn oil, cottonseed oil, cucumber seed oil, flaxseed oil, grape seed oil, hazelnut oil, hemp seed oil, linseed oil, macadamia nut oil, moringa oil, neem oil, olive oil, palm kernel oil, palm oil, peanut oil, pongamia oil, pumpkin seed oil, rice bran oil, safflower oil, sesame oil, soybean oil, sunflower oil, walnut oil and combinations thereof.

5. A composition according to any of the preceding claims, **characterized in that** the total amount of the 2-methoxymethyl-p-phenylenediamine, one of its addition salts, its solvates and/or the solvates of its salts is in a range between 0,001 and 7.5 wt.-% and/or that the total amount of the 2-(1,3-benzodioxol-5-ylamino)ethanol one of its addition salts, its solvates and/or the solvates of its salts, is in a range between 0,001 and 7,5 wt.-% and/or that the total amount of the at least one fatty substance is in a range between 2 and 20 wt.-%.

6. A composition according to any of the preceding claims, **characterized in that** it comprises at least one alkaline agent and/or at least one additional oxidation base and/or at least one additional coupling agent and/or at least one direct dye.

7. A composition according to claim 6, **characterized in that** the total amount of alkaline agent is between 0,5 and 15 wt.-% and/or that the total amount of additional oxidation bases is between 0,001 to 10 wt.-% [description: preferably 0,01 to 8 wt.-%, particularly preferably 0,05 to 5 wt.-%] and/or that the total amount of additional coupling agents is between 0,001 to 10 wt.-% [description: preferably 0,01 to 8 wt.-%, particularly preferably 0,05 to 5 wt.-%] and/or that the total amount of the at least one direct dye is between 0,001 and 5 wt.

8. A composition according to claim 6 or 7, **characterized in that** the alkaline agent is selected from the group consisting of ammonia, an alkanolamine, an amino acid, sodium hydroxide, potassium hydroxide and combinations thereof.

9. A composition according to any of claims 6 to 8, **characterized in that** wherein the at least one additional oxidation base is selected from the group consisting of 1,4-diamino-benzene, 1,4-diamino-2-methyl-benzene, 1,4-diamino-2-(2-hydroxyethyl)-benzene, 1,4-diamino-2-(3-hydroxpropyl)-benzene, 1,4-diamino-2,3-dimethyl-benzene, 1,4-diamino-2,6-dimethylbenzene, 1,4-diamino-2-chloro-benzene, 4-[di(2-hydroxyethyl)amino]-aniline, 2,2'-({2-[(4-aminophenyl)amino]ethyl}imino)diethanol, (4-aminophenyl)-(3-(imidazol-1-yl)propyl)amine, N,N'-bis(β-hydroxyethyl)-N,N'-bis(4'-aminophenyl)-1,3-diaminopropanol, 4-aminophenol, 4-amino-3-methylphenol, bis(5-amino-2-hydroxyphenyl)methane, 1-hydroxyethyl-4,5-diamino pyrazole, 1-hexyl-4,5-diamino pyrazole, 2,3-diaminodihydroxypyrazolo pyrazolone dimethosulfonate, 2,4,5,6-tetraaminopyrimidine and combinations thereof.

10. A composition according to any of claims 6 to 9, **characterized in that** the at least one additional coupling agent is selected from the group consisting of 1,3-Dihydroxy-2-methylbenzene, 3-aminophenol, 5-amino-2-methylphenol, 5-amino-4-chloro-2-methylphenol, 3-amino-2,6-dimethylphenol, 2-methyl-5-hydroxyethylaminophenol, 3-amino-2,4-dichlorophenol, 3,4-dihydro-2H-1,4-benzoxazin-6-ol, 6-amino-3 ,4-dihydro-2H-1,4-benzoxazine, 2,4-diamino-1-(2-hydroxyethoxy)benzene; 2-amino-4-[(2-hydroxyethyl)amino]anisole, 1,3-bis(2,4-diaminophenoxy)propane, 1-methyl-2,6-bis-(2-hydroxyethylamino)-benzene, 2-amino-5-ethylphenol, 1-naphthol, 2-methyl-1-naphthol, 1,5-naphthalenediol, 2,7-naphthalenediol, 2,6-diaminopyridine, 2,6-dihydroxy-3,4-dimethylpyridine, 3,5-diamino-2,6-dimethoxypyridine, 2-amino-3-hydroxypyridine, 6-methoxy-2-methylamino-3-aminopyridine, 3-methyl-1-phenyl-5-pyrazolone, 6-hydroxyindole, 5,6-dihydroxyindole and combinations thereof.

11. A composition according to any of claims 6 to 10, **characterized in that** at least one direct dye is selected from the group consisting of an anionic, a neutral or a cationic dye and combinations thereof.

12. A composition according to any of claims 6 to 11, **characterized in that** at least one direct dye is selected from the group consisting of Acid Yellow 1, Acid orange 7, Acid red 92, Acid violet 43, Tetrabromophenol Blue, 2-Amino-6-chloro-4-nitrophenol, 2-Amino-4-nitrophenol, 2-Amino-5-nitrophenol, Basic Yellow 87, Basic Orange 31, Basic Red 51, Basic Blue 124 and combinations thereof.

13. A composition according to any of the preceding claims, **characterized in that** the composition is free of resorcinol.

14. A multi component kit-of-parts for oxidative dyeing of keratinous fibers, comprising at least two separately packaged components, **characterized in that** a first component comprises a composition according to any of claims 1 to 13 and a second component is an aqueous hydrogen peroxide-containing composition with acidic pH.

15. A process an oxidative dyeing of keratinous fibers, comprising the following steps of
(i) mixing the components of a kit according to claim 14 for yielding a ready-to-use mixture,
(ii) applying the ready-to-use mixture to keratinous fibers,
(iii) leaving the composition on the keratinous fibers for a period of time of 1 to 60 minutes at at least room temperature and
(iv) rinsing the keratinous fibers with water and/or cleaning composition.
